(19) Europäisches Patentamt
European Patent Office
Office européen des brevets

(11) **EP 4 483 889 A1**

## (12) EUROPEAN PATENT APPLICATION

(43) Date of publication:
**01.01.2025 Bulletin 2025/01**

(21) Application number: **23382662.7**

(22) Date of filing: **28.06.2023**

(51) International Patent Classification (IPC):
*A61K 35/74* (2015.01)   *A23L 33/135* (2016.01)
*A61K 9/00* (2006.01)   *A61K 35/742* (2015.01)
*A61K 35/745* (2015.01)   *A61K 35/747* (2015.01)
*A61P 1/02* (2006.01)   *A61Q 11/00* (2006.01)

(52) Cooperative Patent Classification (CPC):
**A61K 35/74; A23L 33/135; A61K 9/0053;
A61K 9/006; A61K 9/06; A61K 9/2018;
A61K 9/205; A61K 35/742; A61K 35/745;
A61K 35/747; A61P 1/02**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Igen Biolab Group AG**
**6300 Zug (CH)**

(72) Inventors:
• MOSCOSO DEL PRADO UCELAY, Juan
28760 Tres Cantos (ES)
• GUARDIA ALBA, María Jesús
18198 Granada (ES)

(74) Representative: **Padial Martinez, Ana Belen**
**Baylos 5.0 Legal Advisors S.L.**
**Avda. Diagonal 435, 1º 1ª**
**08036 Barcelona (ES)**

## (54) POSTBIOTIC COMPOSITION FOR ORAL ADMINISTRATION FOR PREVENTING OR TREATING ORAL DYSBIOSIS

(57) Postbiotic composition of oral administration for use in the treatment and / or prevention of dysbiosis of the oral microbiota in a subject, comprising microorganisms of the following genera: *Bacillus, Bifidobacterium, Lacto-* *bacillus, Saccharomyces* and *Streptococcus,* and which may be presented in the form of toothpaste, mouthwash, bioadhesive gel and / or a soluble solid tablet.

Figure 2

EP 4 483 889 A1

**Description**

**TECHNICAL FIELD OF THE INVENTION**

The present invention is encompassed in the field of oral postbiotic compositions for oral health such as toothpastes, mouthwashes, and soluble solid tablets.

**PRIOR ART**

Oral diseases are a major burden on the health sector in many countries. Treatments are expensive and generally not part of universal health coverage. It is estimated that about 3500 million people are affected worldwide, of which more than 530 million children suffer from dental caries in milk teeth, 10% of the world population is affected by severe periodontitis and consequent tooth loss, and oral cancer affects between 4 to 20 cases per 100,000 people.

The main cause of deterioration is lack of access to oral hygiene products, and inadequate access to oral health care services. In general, these diseases have a multifactorial etiology in which the characteristics of the host, the oral microbiota and the environment in which it develops, such as oral hygiene and diet, intervene. Maintaining a healthy oral microbiota is one of the priorities for healthy oral health.

The oral cavity is one of the most complex regions in terms of microorganisms in the body which are acquired at birth and through food. The various elements that form it such as teeth, buccal and gingivar mucosa, palate or tongue, provide very varied microhabitats with characteristics that favor the development of different niches. In the Human Oral Microbiome Database (HOMD), there are 700 different species that associate themselves forming complex communities which makes it the second largest microbial community in the human body after the intestine. It is composed of mostly harmless bacteria, fungi, viruses, archaea and protozoa, which, after colonization, maintain the stability of the ecosystem, the health of the tissue and of the immune system.

In each niche, nutrients and conditions prevail that favor the proliferation of communities formed by species adapted to live in that microhabitat, whose presence can restrict the invasion or colonization of other species. Commensal microorganisms vary with environmental fluctuations, nutrient availability and type, temperature, pH, oxygen concentration, saliva flow, tissue type, hormonal conditions, and innate immune response. To survive these stress forces and compete with other species for available space, bacteria of the same and / or different species co-aggregate forming biofilms that allow them to survive.

On the surface of the teeth, microorganisms organize themselves becoming more anaerobic in the subgingival plaque than in the supragingival. In soft tissue areas, biofilms are less stable because they have less time to mature. They usually grow in monolayer or in multiple layers that detach easily in the presence of stress. In the tongue, there is a greater quantity and diversity than in other niches. Finally, saliva is formed mainly by microorganisms that are released from biofilms, and contributes to the stability of the ecosystem due to its buffering capacity, being a source of nutrients and antimicrobial compounds.

The dysbiosis of the oral microbiota is the origin of the two main oral pathologies which present a very different profile. Caries is the leading cause of oral pain and tooth loss. Sugar-rich beverages and foods and poor hygiene to remove plaque can lead to bacterial plaque and periodontal disease. Periodontal diseases are all those pathologies that affect the tissues that support the teeth, such as gingivitis and periodontitis. Gingivitis consists of an inflammation of the gums whose aggravation leads to the development of periodontitis and can damage the soft tissues and bones that support the teeth, causing them to fall out. The early inflammation caused in gingivitis can be reversed by oral hygiene and removal of bacterial biofilm. Opportunistic pathogens may exist in persistent plaque that can cause long-term inflammation leading to periodontitis, with irreversible damage to periodontal tissue and alveolar bone.

There are different strategies to modulate the oral microbiota and maintain good oral health. The main one is to maintain a hygiene routine by brushing to control plaque and bacterial load. It is a non-selective method and therefore also acts on beneficial bacteria. The same goes for the use of antibiotics that can modulate the microbiota by acting on both pathogenic and commensal bacteria. These usually contain alcohol, hydrogen peroxide, chlorhexidine, or a mixture of them, and can kill 99% of bacteria as described in Deo PN, Deshmukh R. Oral Microbiome: Unveiling the Fundamentals. J Oral Maxillofac Pathol. 2019;23(1):122. Currently, therapies are being sought that do not generate resistance to microorganisms and that act selectively, favoring oral eubiosis.

Some of these therapies consist of the oral administration of various types of probiotics, but the inventors do not know that any probiotic has been described as having sufficient desirable properties to maintain complete oral health, so a mixture of different strains is necessary to maximize the beneficial effects. In many studies, probiotics fail to colonize because they have to compete with a complex ecosystem with a multitude of interconnections. In the event that this were to happen, the most commonly used probiotics, *Lactobacillus* and *Streptococcus,* divulged in EP3405563, produce acids that can lower pH and can become inducers of new cariogenic lesions. In addition, it is necessary to consider the problem regarding the complexity of maintaining viable probiotics during the production process and formulation of the product to be applied for prevention and / or treatment.

These drawbacks imply that compositions or postbiotic compounds of oral administration that allow maintenance and prevention that is sufficiently effective with respect to oral health are not known in the state of the art.

## DESCRIPTION OF THE INVENTION

In the present report, the term microorganisms includes both bacteria and yeast.

In this report the terms "oral" and "buccal" are synonymous and are used interchangeably.

The terms "treatment" and "treat" refer herein to the medical treatment of a subject with an oral disease with the intention of curing, improving, stabilizing or preventing an aggravation of the disease, pathological condition or disorder. This term includes active treatment, that is, treatment aimed specifically at the improvement of such disease, pathological condition or disorder. In addition, this term includes palliative treatment, i.e. treatment designed to relieve symptoms rather than cure the disease, pathological condition or disorder; preventive treatment, i.e. treatment aimed at minimizing or partially or completely inhibiting the development of the associated disease, pathological condition or disorder; and supportive treatment, i.e. treatment used to complement other specific therapy aimed at improving the associated disease, pathological condition or disorder. It is understood that treatment, although intended to cure, improve, stabilize or prevent a disease, pathological condition or disorder, does not have to actually produce cure, improvement, stabilization or prevention. The effects of treatment may be measured or evaluated as described in this document and as known in the art, as appropriate for the disease, pathological condition or disorder in question. Such measurements and evaluations can be made in qualitative and / or quantitative terms. Thus, for example, the characteristics or traits of a disease, pathological condition or disorder and / or the symptoms of a disease, pathological condition or disorder can be reduced to any effect or in any quantity. In the context of a subject suffering from an oral disease, the terms "treatment" and "treat" refer to the medical management of a subject with the intention of curing, improving, or stabilizing said disease, as well as improving the symptoms associated with this disease. Based on this, what has been developed is a composition that is administered orally, comprising lysates of microorganisms that can be accompanied by other components for this composition, and which has turned out to be strikingly beneficial for humans and animals.

The present invention is intended to overcome or at least mitigate the drawbacks of the prior art by means of an oral postbiotic composition for use in the treatment and / or prevention of oral microbiota dysbiosis in a subject, comprising a mixture of lysates of probiotic microorganisms in an amount in percentage by weight with respect to the total weight of the mixture of lysates of microorganisms of the composition:

- 6% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces*;
- 1.5% to 7% of bacterial lysates of the genus *Streptococcus*.

In a particular embodiment, the postbiotic composition of oral administration for use in the treatment and / or prevention of oral diseases comprising a mixture of lysates of probiotic microorganisms in an amount in percentage by weight with respect to the total weight of the mixture of lysates of microorganisms of the composition:

- 13% to 19% of bacterial lysates of the genus *Bacillus*;
- 4% to 8% of bacterial lysates of the genus *Bifidobacterium*;
- 15% to 25% of bacterial lysates of the genus *Lactobacillus*;
- 50% to 60% of yeast lysates of the genus *Saccharomyces*;
- 1.5% to 5% of bacterial lysates of the genus *Streptococcus*.

In a particular embodiment, the percentage of *Lactobacillus* and *Streptococcus* lysates does not exceed 30% of the total weight, preferably 25% of the total weight.

In accordance with the invention, the composition can comprise between 1%-99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%±10% by weight of lysates of microorganisms.

The composition of the invention may comprise additional components or additives, or any other substance such as salts or excipients, that facilitate the packaging, preparation and / or administration of the composition but do not affect the ability to treat oral diseases of the postbiotic composition of the invention.

In the context of the present invention, lysates of probiotic microorganisms are understood to be the product obtained after the process of cultivating, and subsequently mechanically or chemically breaking up said cells in order to obtain a product with microbial fragments, as well as all the components that are comprised therein. Moreover, in this document it is indicated that these are lysates of dry probiotic microorganisms, since, due to their method of obtainment, these lysates are subsequently subjected to drying techniques, in such a way that said dry lysates are optionally found in the form of dry powder.

In preferred embodiments of the present invention, the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus, Bacillus paralicheniformes,* and combinations thereof. Preferably, *Bacillus licheniformis, Bacillus pumilus* and *Bacillus subtilis.*

In other embodiments, the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof. Preferably, *Bifidobacterium animalis subsp lactis* and *Bifidobacterium lactis.*

In other embodiments, the bacterial lysates of the genus *Lactobacillus* are of the species that are selected from the group consisting of *Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helveticus, Lactobacillus gasseri, Lactobacillus johnsonii, Lactobacillus brevis, Lactobacillus kefiri,* and combinations thereof. Preferably, *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius.*

In other particular embodiments of the present invention, the lysates of yeasts of the genus *Saccharomyces* are of the species that are selected from the group consisting of *Saccharomyces cerevisiae, Saccharomyces boulardi,* and combinations thereof, preferably *Saccharomyces cerevisiae.*

In other particular embodiments of the present invention, the bacterial lysates of the genus *Streptococcus* are of the species that are selected from the group consisting of *Streptococcus thermophilus, Streptococcus salivarius,* and combinations thereof, preferably *Streptococcus thermophilus.*

In preferred embodiments of the present invention, the composition object of the invention comprises bacterial lysates of the genus *Bacillus* in an amount in percentage by weight between approximately 6% to 19% of the total lysates of the composition, preferably 13% to 19% of the total lysates of the composition. In preferred embodiments, the amount of bacterial lysates of the genus *Bacillus* can be approximately 14%, 15%, 16%, 17%, or 18% of the total lysates of the composition.

In a particular embodiment, the percentage by weight of *Bacillus licheniformis, Bacillus pumilus* and *Bacillus subtilis* is approximately the same.

In preferred embodiments of the present invention, the composition object of the invention comprises bacterial lysates of the genus *Bifidobacterium* in an amount in percentage by weight between approximately 4% to 8% of the total lysates of the composition, preferably between 5% to 7%. In preferred embodiments, the amount of bacterial lysates of the genus *Bifidobacterium* can be approximately 5%, 6%, 7%, or 8% of the total lysates of the composition.

In a particular embodiment, the percentage by weight of *Bifidobacterium lactis* is higher than the percentage by weight of *Bifidobacterium animalis subsp lactis.*

In preferred embodiments of the present invention, the composition object of the invention comprises bacterial lysates of the genus *Lactobacillus* in an amount in percentage by weight between approximately 15% to 25% of the total lysates of the composition, and preferably between 20% to 24% on the total lysates of the composition. In preferred embodiments, the amount of bacterial lysates of the genus *Lactobacillus* can be about 20%, 21%, 22%, 23%, 24%, or 25% of the total lysates of the composition.

In a particular embodiment, when the bacterial lysates of the genus *Lactobacillus* are of *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius,* the percentage by weight of *Lactobacillus acidophilus* and *Lactobacillus casei* is different, the percentage by weight of Lactobacillus *reuteri* and *Lactobacillus rhamnosus* being different from the aformentioned but identical between themselves, the percentage by weight of *Lactobacillus paracasei* and *Lactobacillus salivarius* being different from the aformentioned but identical between themselves, the percentage by weight of *Lactobacillus fermentum* and *Lactobacillus plantarum* being different from the aforementioned but identical between themselves.

In another particular embodiment, when the bacterial lysates of the genus *Lactobacillus* are *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* the percentage by weight of *Lactobacillus casei* is superior to the rest of the *Lactobacillus* species.

In another particular embodiment, when the bacterial lysates of the genus *Lactobacillus* are *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* the percentage by weight of *Lactobacillus casei* is superior to that of *Lactobacillus acidophilus,* which in turn is superior to that of *Lactobacillus reuteri* and *Lactobacillus*

*rhamnosus,* the percentage by weight of these 2 species being approximately identical, which in turn is higher than that of *Lactobacillus fermentum* and *Lactobacillus plantarum,* the percentage by weight of these 2 species being approximately identical, which in turn is higher than that of *Lactobacillus paracasei* and *Lactobacillus salivarius,* the percentage by weight of these 2 species being approximately identical.

In preferred embodiments of the present invention, the composition comprises lysates of yeast of the genus *Saccharomyces* in an amount in percentage by weight between approximately 50% to 60% of the total lysates of the composition, preferably between 52% to 57% of the total lysates of the composition. In preferred embodiments, the amount of lysates of yeast of the genus *Saccharomyces* can be about 50%, 51%, 52%, 53%, 54%, 55%, 56%, 57%, 58%, 59%, or 60% of the total lysates of the composition.

In preferred embodiments of the present invention, the composition comprises bacterial lysates of the genus *Streptococcus* in an amount in percentage by weight between about 1.5% to 5% of the total lysates of the composition, preferably between about 3% to 4% of the total lysates of the composition. In preferred embodiments, the amount of bacterial lysates of the genus *Streptococcus* can be about 1.5%, 1.6%, 1.7%, 1.8%, 1.9%, 2%, 2.1%, 2.2%, 2.3%, 2.4%, 2.5%, 2.6%, 2.7%, 2.8%, 2.9%, or 3% of the total lysates of the composition. In other preferred embodiments, the amount of bacterial lysates of the genus *Streptococcus* can be about 3.5%, 4%, 4.5%, or 5% of the total lysates of the composition.

In a particular embodiment of the invention, the composition comprises lysates of probiotic microorganisms in an amount in percentage by weight on the total lysates of the composition:

- approximately 13% to 17% of bacterial lysates of the genus *Bacillus;*
- approximately 4.5% to 7.5% of bacterial lysates of the genus *Bifidobacterium;*
- approximately 20% to 22% of bacterial lysates of the genus *Lactobacillus;*
- approximately 53% to 57% of yeast lysates of the genus *Saccharomyces;*
- approximately 2.5% to 3.5% of bacterial lysates of the genus *Streptococcus.*

This composition is called composition "A".

In another particular embodiment of the invention, the composition comprises lysates of probiotic microorganisms in an amount in percentage by weight on the total lysates of the composition:

- approximately 15% to 17% of bacterial lysates of the genus *Bacillus;*
- approximately 4.6% to 6.5% of bacterial lysates of the genus *Bifidobacterium;*
- approximately 20.5% to 21.8% of bacterial lysates of the genus *Lactobacillus;*
- approximately 54% to 56% of yeast lysates of the genus *Saccharomyces;*
- approximately 2.8% to 3.2% of bacterial lysates of the genus *Streptococcus.*

This composition is called composition "B".

In another particular embodiment of the invention, the composition comprises lysates of probiotic microorganisms in an amount in percentage by weight with respect to the total:

- approximately 6% to 19% of bacterial lysates of *Bacillus licheniformis, Bacillus pumilus* and *Bacillus subtilis;*
- approximately 4% to 8% of bacterial lysates of *Bifidobacterium animalis subsp lactis* and *Bifidobacterium lactis;*
- approximately 15% to 25% of bacterial lysates of *Lactobacillus acidophilus, Lactobacillus casei, Lactobacillus fermentum, Lactobacillus paracasei, Lactobacillus plantarum, Lactobacillus reuteri, Lactobacillus rhamnosus* and *Lactobacillus salivarius;*
- approximately 50% to 60% of yeast lysates of *Saccharomyces cerevisiae;*
- approximately 1.5% to 7% of bacterial lysates of *Streptococcus thermophilus.*

This composition is called composition "C".

In another particular embodiment of the invention, the composition comprises lysates of probiotic microorganisms in an amount in percentage by weight with respect to the total:

- approximately 13% to 19% of bacterial lysates of *Bacillus licheniformis, Bacillus pumilus* and *Bacillus subtilis;*
- approximately 4% to 8% of bacterial lysates of *Bifidobacterium animalis subsp lactis* and *Bifidobacterium lactis;*

- approximately 15% to 25% of bacterial lysates of *Lactobacillus acidophilus, Lactobacillus casei, Lactobacillus fermentum, Lactobacillus paracasei, Lactobacillus plantarum, Lactobacillus reuteri, Lactobacillus rhamnosus* and *Lactobacillus salivarius;*
- approximately 50% to 60% of yeast lysates of *Saccharomyces cerevisiae;*
- approximately 1.5% to 5% of bacterial lysates of *Streptococcus thermophilus.*

This composition is called composition "D".

In another particular embodiment of the invention, the composition comprises lysates of probiotic microorganisms in an amount in percentage by weight with respect to the total:

- approximately 13% to 17% of bacterial lysates of *Bacillus licheniformis, Bacillus pumilus* and *Bacillus subtilis;*
- approximately 4.5% to 7.5% of bacterial lysates of *Bifidobacterium animalis subsp lactis* and *Bifidobacterium lactis;*
- approximately 20% to 22% of bacterial lysates of *Lactobacillus acidophilus, Lactobacillus casei, Lactobacillus fermentum, Lactobacillus paracasei, Lactobacillus plantarum, Lactobacillus reuteri, Lactobacillus rhamnosus* and *Lactobacillus salivarius;*
- approximately 53% to 57% of yeast lysates of *Saccharomyces cerevisiae;*
- approximately 2.5% to 3.5% of bacterial lysates of *Streptococcus thermophilus.*

This composition is called composition "E".

In another particular embodiment of the invention, the composition comprises lysates of probiotic microorganisms in an amount in percentage by weight with respect to the total:

- approximately 15% to 17% bacterial lysates of *Bacillus licheniformis, Bacillus pumilus* and *Bacillus subtilis;*
- approximately 4.6% to 6.5% of bacterial lysates of *Bifidobacterium animalis subsp lactis* and *Bifidobacterium lactis;*
- approximately 20.5% to 21.8% of bacterial lysates of *Lactobacillus acidophilus, Lactobacillus casei, Lactobacillus fermentum, Lactobacillus paracasei, Lactobacillus plantarum, Lactobacillus reuteri, Lactobacillus rhamnosus* and *Lactobacillus salivarius;*
- approximately 54% to 56% of yeast lysates of *Saccharomyces cerevisiae;*
- approximately 2.8% to 3.2% of bacterial lysates of *Streptococcus thermophilus.*

This composition is called composition "F".

In another particular embodiment of the invention, the composition comprises lysates of probiotic microorganisms in an amount in percentage by weight with respect to the total:

- approximately 6% to 19% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;
- approximately 4% to 8% of bacterial lysates of *Bifidobacterium animalis subsp lactis* and *Bifidobacterium lactis,* the percentage by weight of *Bifidobacterium lactis* being higher than the percentage by weight of *Bifidobacterium animalis subsp lactis;*
- approximately 15% to 25% of bacterial lysates of *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius,* the percentage by weight of *Lactobacillus casei* being higher than that of *Lactobacillus acidophilus,* the percentage by weight of *Lactobacillus reuteri* and *Lactobacillus rhamnosus* being lower than that of *L. casei and L. acidophilus,* but approximately the same between themselves, the percentage by weight of *Lactobacillus paracasei* and *Lactobacillus salivarius* being lower than that of the four aforementioned species but approximately the same between themselves, the percentage by weight of *Lactobacillus fermentum* and *Lactobacillus plantarum* being lower than that of the six aforementioned species but approximately the same between themselves.
- approximately 50% to 60% of yeast lysates of *Saccharomyces cerevisiae;*
- approximately 1.5% to 7% of bacterial lysates of *Streptococcus thermophilus.*

This composition is called composition "G".

In another particular embodiment of the invention, the composition comprises lysates of probiotic microorganisms in an amount in percentage by weight with respect to the total:

- approximately 13% to 19% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same.
- approximately 4% to 8% of bacterial lysates of *Bifidobacterium animalis subsp lactis* and *Bifidobacterium lactis,* the percentage by weight of *Bifidobacterium lactis* being higher than the percentage by weight of *Bifidobacterium animalis subsp lactis:*
- approximately 15% to 25% of bacterial lysates of *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* the percentage by weight of *Lactobacillus casei* being higher than that of *Lactobacillus acidophilus,* the percentage by weight of *Lactobacillus reuteri* and *Lactobacillus rhamnosus* being lower than that of *L. casei and L. acidophilus,* but approximately the same between themselves, the percentage by weight of *Lactobacillus*

*paracasei* and *Lactobacillus salivarius* being lower than that of the four aforementioned species but approximately the same between themselves, the percentage by weight of *Lactobacillus fermentum* and *Lactobacillus plantarum* being lower than that of the six aforementioned species but approximately the same between themselves;

- approximately 50% to 60% of yeast lysates of *Saccharomyces cerevisiae;*
- approximately 1.5% to 5% of bacterial lysates of *Streptococcus thermophilus.*

This composition is called composition "H".

In another particular embodiment of the invention, the composition comprises lysates of probiotic microorganisms in an amount in percentage by weight with respect to the total:

- approximately 13% to 17% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;
- approximately 4.5% to 7.5% of bacterial lysates of *Bifidobacterium animalis subsp lactis* and *Bifidobacterium lactis,* the percentage by weight of *Bifidobacterium lactis* being higher than the percentage by weight of *Bifidobacterium animalis subsp lactis;*
- approximately 20% to 22% of bacterial lysates of *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* the percentage by weight of *Lactobacillus casei* being higher than that of *Lactobacillus acidophilus,* the percentage by weight of *Lactobacillus reuteri* and *Lactobacillus rhamnosus* being lower than that of *L. casei* and *L. acidophilus* but approximately the same between themselves, the percentage by weight of *Lactobacillus paracasei* and *Lactobacillus salivarius* being lower than that of the four aforementioned species but approximately the same between themselves, the percentage by weight of *Lactobacillus fermentum* and *Lactobacillus plantarum* being lower than that of the six aforementioned species but approximately the same between themselves;
- approximately 53% to 57% of yeast lysates of *Saccharomyces cerevisiae;*
- approximately 2.5% to 3.5% of bacterial lysates of *Streptococcus thermophilus.*

This composition is called composition "I".

In another particular embodiment of the invention, the composition comprises lysates of probiotic microorganisms in an amount in percentage by weight with respect to the total:

- approximately 15% to 17% of bacterial lysates of *Bacillus licheniformis, Bacillus pumilus* and *Bacillus subtilis,* the percentage by weight of *Bacillus licheniformis, Bacillus pumilus* and *Bacillus subtilis* being approximately the same;
- approximately 4.6% to 6.5% of bacterial lysates of *Bifidobacterium animalis subsp lactis* and *Bifidobacterium lactis,* the percentage by weight of *Bifidobacterium lactis* being higher than the percentage by weight of *Bifidobacterium animalis subsp lactis;*
- approximately 20.5% to 21.8% of bacterial lysates of *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* the percentage by weight of *Lactobacillus casei* being higher than that of *Lactobacillus acidophilus,* the percentage by weight of *Lactobacillus reuteri* and *Lactobacillus rhamnosus* being lower than that of *L. casei* and *L. acidophilus* but approximately the same between themselves, the percentage by weight of *Lactobacillus paracasei* and *Lactobacillus salivarius* being lower than that of the four aforementioned species but approximately the same between themselves, the percentage by weight of *Lactobacillus fermentum* and *Lactobacillus plantarum* being lower than that of the six aforementioned species but approximately the same between themselves;
- approximately 54% to 56% of yeast lysates of *Saccharomyces cerevisiae;*
- approximately 2.8% to 3.2% of bacterial lysates of *Streptococcus thermophilus.*

This composition is called composition "J".

In another particular embodiment, in any of the compositions of the invention, including any of the compositions A to J, the percentage by total weight of the bacterial lysates of the species of *Lactobacillus* and *Streptococcus* is equal to or less than about 30%, preferably equal to or less than about 24%.

The postbiotic composition of the invention, being composed of lysates of microorganisms of multiple probiotic species is intended to control or at least mitigate oral health disorders caused by the dysbiosis of the oral microbiota, such as dental caries, gingivitis, periodontitis, halitosis, as well as to control the translocation of microorganisms and / or their components (proteins, nucleic acids) that can generate diseases in places distant from the oral cavity. In addition, it avoids, unlike compositions comprising probiotics, the possible colonization by these microorganisms as well as the high cost of keeping them viable during the preparation of the compounds.

In a particular embodiment, dysbiosis of the oral microbiota produces dental caries.

In another particular embodiment, dysbiosis of the oral microbiota, preferably dental caries, is produced, at least partially, by S. *mutans.*

In another particular embodiment, dysbiosis of the oral microbiota produces periodontitis.

In another particular embodiment, dysbiosis of the oral microbiota, preferably periodontitis, is produced, at least partially, by one or more of the following: *A. actinomycetemcomitans,* P. *intermedia* and *P.gingivalis.*

The postbiotic composition comprising microorganisms of the genera *Lactobacillus sp., Bifidobacterium sp., Bacillus sp., Streptococcus sp.,* and *Saccharomyces sp.,* with an immunomodulatory effect that can act systemically in addition to having an inhibitory capacity for the growth of pathogens associated with oral diseases that would modulate the oral microbiota.

In particular embodiments of the present invention, the compositions object of the invention, are obtained by a method comprising the following steps:

- Cultivate the microbial species selected for the preparation of the composition using the standard conditions established for the microbial species listed in this document;
- Filter or centrifuge the culture until an adequate biomass of the selected microorganisms is obtained;
- Lyse by freezing and thawing cycles and sonication of the resulting biomasses to obtain at least 90% or 91% or 92% or 94% of bacterial lysates, preferably at least 95% or 96% or 97% or 98%, and most preferably, 99% or 100%;
- Mix the different strains in the proportions selected according to the percentage by weight to obtain the composition of lysates of probiotic microorganisms of the invention.

A skilled person would know how to find the appropriate protocol for the growth of the microorganisms of the composition, as well as the protocol for their lysis since these are common procedures in the state of the art.

In a particular embodiment, the method of obtaining the composition of the invention comprises an additional step: Dry by atomization or lyophilization the biomasses of bacterial lysates obtaining a product in powder form comprising all the bacterial strains selected to prepare the composition object of the invention.

In a particular embodiment, the composition object of the invention is presented in the form of dry powder.

When the composition object of the invention is presented in the form of dry powder, it is necessary to dissolve it in water or any other liquid that does not affect the properties of the components of the composition prior to its intake by the patient. The advantages of dry powder are that it is easier to transport and store, and also increases the stability and durability of the composition. In the present report, dry powder means that the humidity level is less than 10%, preferably less than 5%, more preferably less than 1%.

In particular embodiments, the composition object of the invention is obtained from cultures of probiotic microorganisms of the genera described in the present document, which comprise a certain amount of Colony Forming Units (CFUs). In the context of the present invention, a Colony Forming Unit is a term of microbiology. It is an indicator of the amount of live microorganisms present in a medium.

The process of obtaining lysates comprises a heat treatment alternating heating and cooling cycles. Each batch of viable cell culture is resuspended in distilled water in a ratio of approximately 1: 1 and subjected to a sterilization cycle in an autoclave at 121°C for 20 minutes. They are then frozen at -20°C for 12 hours and thawed at room temperature the next day.

Once thawed, they undergo a cell rupture treatment by sonication for 20min, at an output power of 500 W at an amplitude of 40% and 10 seconds of pause, obtaining a mass of lysed cells from the probiotic batch (Qsonica, Q500). The final solution is freeze-dried and ground to obtain a lysate of the powdery probiotic microorganism. The powder is kept in a cool environment away from heat. The degree of survival in no case exceeds a final viability approximately 7 E3 cfu/ml, which implies practically 100% cell death.

In particular embodiments of the present invention, one starts from a quantity of bacteria of the genus *Bacillus* comprising between 3.49% to 20.94% of CFU with respect to the total CFU of the composition, most preferably between approximately 10.02% to 15.75%. In absolute values, the culture grown, before proceeding to the lysis of the cells, presents between 1 E+11 and 5E+11 cells, preferably between 2E+11 and 3E+11, more preferably approximately 2.75E+11.

In another particular embodiment, when the species of the genus *Bacillus* are: *Bacillus licheniformis, Bacillus mesentericus* and *Bacillus subtilis,* each of the grown cultures presents, before proceeding to the lysis of the cells, between 9E+10 and 3E+11 cells of *Bacillus licheniformis,* between 6E+10 and 9E+10 cells of *Bacillus mesentericus* and between 9E+10 and 3E+11 cells of *Bacillus subtilis.*

In another embodiment, one starts with a number of bacteria of the genus *Bifidobacterium* comprising between 8.20% to 53.64% of CFU with respect to the total culture, most preferably between 9.99% to 18.75%. In absolute values, the culture grown, before proceeding to the lysis of the cells, presents between 1E+11 and 7E+11 cells, preferably between 2E+11 and 5 E+11, more preferably approximately 2.70E+11.

In another particular embodiment, when the species of the genus *Bifidobacterium* are: *Bifidobacterium animalis subsp lactis* and *Bifidobacterium lactis,* each of the grown cultures presents, before proceeding to the lysis of the cells, between 1E+10 and 5E+10 cells of *Bifidobacterium animalis subsp lactis,* and between 3E+11 and 5E+9 cells of *Bifidobacterium lactis.*

In particular embodiments of the present invention, one starts with a number of bacteria of the genus *Lactobacillus* comprising between 28.64% to 66.82% of CFU with respect to the total CFU of the composition, more preferably between 40.66% to 57.36%. In absolute values, the culture grown, before proceeding to the lysis of the cells presents between 8E+11 and 3E+12 cells, preferably between 9E+11 and 2E+12, more preferably approximately 1.22E+12.

In another particular embodiment, when the species of the genus *Lactobacillus* are: *Lactobacillus acidophilus, Lactobacillus casei, Lactobacillus paracasei, Lactobacillus plantarum, Lactobacillus fermentum Lactobacillus reuteri, Lactobacillus rhamnosus* and *Lactobacillus salivarius,* each of the grown cultures presents, before proceeding to the lysis of the cells, between 5E+10 and 9E+10 cells of *Lactobacillus acidophilus,* between 2E+11 and 8E+11 cells of *Lactobacillus casei,* between 3E+10 and 9E+10 cells of *Lactobacillus fermentum,* between 9E+9 and 4E+10 cells of *Lactobacillus paracasei,* between 9E+10 and 4E+11 cells of *Lactobacillus plantarum,* between 3E+10 and 9E+10 cells of *Lactobacillus reuteri,* between 1 E+11 and 6E+11 cells of *Lactobacillus rhamnosus* and between 9E+9 and 4E+10 cells of *Lactobacillus salivarius.*

In particular embodiments of the present invention, one starts with a number of bacteria of the genus *Streptococcus* comprising between 5.72% to 38.15% of CFU with respect to the total CFU of the composition, most preferably between 10.98% to 20.75%. In absolute values, the culture grown, before proceeding to the lysis of the cells presents between 1E+11 and 6E+11 cells, preferably between 2E+11 and 4E+11, more preferably approximately 3.00E+11. In a particular embodiment the species of *Streptococcus* is *Streptococcus thermophilus.*

In particular embodiments of the present invention, one starts with a quantity of bacteria of the genus *Saccharomyces* comprising between 11.44% to 24.79% of CFU with respect to the total culture, most preferably between 19.04% to 21.75%. In absolute values, the culture grown, before proceeding to the lysis of the cells presents between 1 E+11 and 7E+11 cells, preferably between 4E+11 and 6E+11, more preferably approximately 5,20E+11. In a particular embodiment the species of *Saccharomyces* is *Saccharomyces cerevisie.*

These probiotic microorganisms are cultivated under standard conditions indicated for each of the species described in this document, as set out in the culture protocols published by the Spanish Collection of Type Cultures (CECT).

Once these microorganisms are cultured, they are subjected to a lysis procedure. First of all, the microbial cells undergo heat treatment. Each batch of viable cell culture undergoes a sterilization cycle in an autoclave at 121°C for 20 to 30 minutes. This temperature denatures and coagulates the proteins by inactivating them. In addition, it causes damage to membranes, aggregation of ribosomes, breaking of DNA strands and inactivation of enzymes. Once cold, they undergo a cell rupture treatment by sonication for a period of 15 to 20min, at an output power of between 450 to 550 W at an amplitude of between 38 to 43% and a period of 8 to 15 seconds of pause, obtaining a mass of lysed cells from the probiotic batch (Qsonica, Q500). This results in the breakage of intermolecular interactions and DNA fragmentation. Optionally, the final solution is freeze-dried and ground to obtain a lysate of the powdery probiotic microorganism. The powder is stored in a cool, dry environment away from heat.

The composition in accordance with the invention is the result of the combination of probiotic microorganisms that after a process of growth and lysis generates an extract consisting of a set of metabolites, proteins, DNA fragments and other components, such as, for example, peptidoglycans, which dosed effectively is able to alter and modify in a striking way the microbiota of the host through several mechanisms.

In a particular embodiment, when the composition object of the invention is in powder form, the composition, in accordance with the first aspect of the invention, can be presented in sealed sachets, in themselves conventional in the food and pharmaceutical industry.

Another form of presentation of the food supplement, in accordance with the invention, is in capsule form, such as conventional gelatin capsules, inside which the composition is contained in powder form.

In another particular embodiment, the dysbiosis of the oral microbiota comprises one or more of the following conditions: dental caries, gingivitis, periodontitis, halitosis and other diseases of the oral cavity.

In another particular embodiment, oral hygiene includes reducing the proportions of Gram-negative anaerobic genera and / or increasing the proportions of aerobic or facultative anaerobic genera in the oral cavity, reaching the usual situation that is the presence of more anaerobic microorganisms in the subgingival plaque than in the supragingival.

Thus, in the context of the present invention, it has been determined that at least one dose has to be administered, preferably at least one daily dose, between 100mg to 80000mg of the composition of the invention including any of the compositions A-J. In preferred embodiments, a dose may comprise between 100mg to 50000mg of the composition of the invention including any of the compositions A-J, in another preferred embodiment a dose may comprise between 100mg to 2000mg of the composition of the invention including any of the compositions A-J, in another preferred embodiment a dose may comprise between 100mg to 700mg of the composition of the invention including any of the compositions A-J. In particular embodiments of the present invention, a dose may comprise approximately 120mg, 130mg, 140mg, 150mg, 160mg, 170mg, 180mg, 190mg, 200mg 210mg, 220mg, 230mg, 240mg, 250mg, 260mg, 270mg, 280mg, 290mg, 300mg 310mg, 320mg, 330mg, 340mg, 350mg, 360mg, 370mg, 380mg, 390mg, 400mg, 410mg, 420mg, 430mg, 440mg, 450mg, 460mg, 470mg, 480mg, 490mg, 500mg, 510mg, 520mg, 530mg, 540mg, 550mg, 560mg, 570mg, 580mg, 590mg, 600mg, 610mg, 620mg, 630mg, 640mg, 650mg, 660mg, 670mg, 680mg, or 690mg of the composition object of the invention including any of the compositions A-J.

In a particular embodiment, you can administer at least one dose of the composition object of the invention, including any of the compositions A-J, daily, or every other day, or every 3 or 4 days, following any of the concentrations of the preceding paragraph.

In another additional embodiment, the administration of the postbiotic composition of the invention including any of the compositions A-J is daily for at least 2 weeks, preferably daily for at least 3 weeks, more preferably daily for at least 4 weeks, even more preferably daily for at least 5 weeks, even more preferably daily for at least 6 weeks, even more preferably daily for at least 7 weeks, even more preferably daily for at least 8 weeks, or daily for at least 9 weeks, or daily for at least 10 weeks, or daily for at least 11 weeks, or daily for at least 12 weeks.

Preferably the composition object of the invention, including any of the compositions A-J, is administered in an amount between 100mg to 700mg per dose and between 1 to 6 doses per day for at least 2 weeks.

An additional object of the invention relates to a pharmaceutical composition comprising an effective pharmaceutical amount of the composition, in accordance with the first aspect of the invention, and a pharmaceutically acceptable excipient.

In the context of the present invention, the expression "pharmaceutical composition" refers to a formulation that has been adapted to deliver a predetermined dose of one or more useful therapeutic agents to a cell, a group of cells, an organ, or a tissue.

The expression "effective pharmaceutical quantity", as used herein, is understood as a nontoxic amount capable of providing a therapeutic effect, the exact amount required will vary from one subject to another, depending on the species, age and general condition of the subject, the severity of the disease being treated, the particular compound used, its mode of administration, and the like. Therefore, it is not possible to specify an exact "effective quantity". However, an appropriate effective amount can be determined by a skilled person in the domain through routine experimentation. In the context of compounds and compositions for the prevention of infection, the pharmaceutically effective amount can refer to the amount needed to alleviate the symptoms of dysbiosis of the oral microbiota.

Moreover, in the context of the present invention, "pharmaceutically acceptable excipient" means a therapeutically inactive substance that is said to be used to incorporate the active ingredient and that is acceptable to the patient from a pharmacological / toxicological standpoint and to the pharmaceutical chemist who manufactures it from a physical / chemical standpoint with respect to composition, formulation, stability, patient acceptance and bioavailability.

The pharmaceutical composition of the invention may be presented in the form of toothpaste, bioadhesive gel, mouthwash and / or soluble solid tablet.

When the composition, in accordance with the invention, is a bioadhesive gel, it comprises:

0.2-40% by weight of the postbiotic composition of the invention and

99.8 -60% by weight of at least one excipient.

In a particular embodiment, the excipients of the bioadhesive gel are Hyaluronic acid at approximately 0.2% by weight, Chlorhexidine at approximately 0.2% by weight, and Panthenol at approximately 5% by weight.

In another particular embodiment, the bioadhesive gel is Perio·aid® bioadhesive gel from the company DENTAID. This bioadhesive gel comprises the excipients indicated in the previous paragraph in the same percentage by weight.

In another particular embodiment, the composition of the bioadhesive gel is any of those described in WO2019025599A1.

An additional object of the invention relates to a bioadhesive gel comprising the postbiotic composition described above.

When the composition, according to the invention, is a toothpaste, it comprises:

0.2-40% by weight of the postbiotic composition of the invention and

99.8-60% by weight of at least one excipient.

In a particular embodiment, the toothpaste of the invention comprises approximately 0.3% by weight of the postbiotic composition of the invention and approximately 99.7% by weight of at least one excipient, particularly approximately 1% by weight of the postbiotic composition of the invention and approximately 99% by weight of at least one excipient, particularly approximately 5% by weight of the postbiotic composition of the invention and approximately 95% by weight of at least one excipient, particularly approximately 10% by weight of the postbiotic composition of the invention and approximately 90% by weight of at least one excipient, particularly approximately 15% by weight of the postbiotic composition of the invention and approximately 85% by weight of at least one excipient, particularly approximately 20% by weight of the postbiotic composition of the invention and approximately 80% by weight of at least one excipient, particularly approximately 25% by weight of the postbiotic composition of the invention and approximately 75% by weight of at least one excipient, particularly approximately 30% by weight of the postbiotic composition of the invention and approximately 70% by weight of at least one excipient, particularly approximately 35% by weight of the postbiotic composition of the invention and approximately 65% by weight of at least one excipient, particularly approximately 40% by weight of the postbiotic composition of the invention and approximately 60% by weight of at least one excipient.

In another particular embodiment, the toothpaste of the invention comprises between approximately 5-20% by weight of the postbiotic composition of the invention and approximately 95-80% by weight of at least one excipient.

The excipient of the toothpaste can be selected from preservatives, flavorings, aromas, binders, foaming agents, humectants, abrasives, water, and combinations thereof.

Toothpaste can comprise 20-60% by weight of the humectant. The humectant can be selected from glycerin, sorbitol, and combinations thereof.

Toothpaste can comprise 30-99.8% by weight of the abrasive. The abrasive can be selected from dicalcium phosphate, hydrated dental silica, and combinations thereof.

In a particular embodiment of the toothpaste, it includes the excipients and composition according to Table 1:

Table 1: Example of toothpaste composition

| Component | % by weight |
|---|---|
| sodium benzoate | 0,1 - 0,5 % |
| sodium saccharin | 1 - 2 % |
| lemon oil | 1 - 2 % |
| cellulose gum | 1 - 2% |
| sodium lauryl sulfate | 1 - 3 % |
| glycerine | 10 - 30% |
| sorbitol | 10 - 30% |
| dicalcium phosphate | 15 - 50 % |
| hydrated dental silica | 15 - 50 % |
| Composition of the invention | 0.2 - 40 % |

Water is a carrier ingredient that moisturizes and dissolves many ingredients and serves to confer the necessary viscosity to toothpaste. In a particular embodiment, the composition of Table 1 comprises water up to 100% by weight.

Humectants prevent toothpaste from drying out and maintain its moisture during storage. Glycerin and sorbitol maintain the viscosity of the toothpaste preventing the product from drying out.

Hydrated dental silica and dicalcium phosphate are abrasives that help clean, polish and remove surface stains, while protecting tooth enamel. Silica removes surface stains when combined with the mechanical action of the toothbrush. It is incorporated into the composition with the purpose of facilitating the mechanical brushing of the toothbrush and reducing the time needed for the cleaning of the tooth surface.

Sodium lauryl sulfate is a foaming and cleaning agent that helps shed food particles, bacteria, and plaque, and helps remove stains from teeth. It helps to create a stable suspension in the mouth which allows an effective cleaning.

Cellulose gum is a binder that increases the viscosity of the toothpaste, providing consistency to the formulation.

Sodium saccharin and lemon oil flavor the toothpaste.

Sodium benzoate is a preservative that is added to protect the toothpaste from the effects of microorganisms that can alter its properties.

An additional object of the invention relates to a toothpaste comprising the postbiotic composition described above.

When the composition, in accordance with the invention, is a mouthwash or liquid mixture, this mouthwash or liquid mixture comprises:

0.2-40% by weight of the postbiotic composition of the invention and

1-40% by weight of at least one excipient;

In a particular embodiment, the mouthwash of the invention comprises approximately 0.3% by weight of the postbiotic composition of the invention and approximately 40% by weight of at least one excipient, particularly approximately 1% by weight of the postbiotic composition of the invention and approximately 35% by weight of at least one excipient, particularly approximately 5% by weight of the postbiotic composition of the invention and approximately 30% by weight of at least one excipient, particularly approximately 10% by weight of the postbiotic composition of the invention and approximately 25% by weight of at least one excipient, particularly approximately 15% by weight of the postbiotic composition of the invention and approximately 20% by weight of at least one excipient, particularly approximately 20% by weight of the postbiotic composition of the invention and approximately 15% by weight of at least one excipient, particularly approximately 25% by weight of the postbiotic composition of the invention and approximately 10% by weight of at least one excipient, particularly approximately 30% by weight of the postbiotic composition of the invention and approximately 5% by weight of at least one excipient, particularly approximately 35% by weight of the postbiotic composition of the invention and approximately 3% by weight of at least one excipient, particularly approximately 40% by weight of the postbiotic composition of the invention and approximately 1% by weight of at least one excipient.

In another particular embodiment, the mouthwash of the invention comprises between approximately 5-20% by weight of the postbiotic composition of the invention and approximately 5-30% by weight of at least one excipient.

The excipient of the mouthwash may be selected from sweeteners, flavorings, anti-caking agents, and combinations thereof. The solvent of the liquid mixture or mouthwash is water or any other liquid that does not affect the properties of the postbiotic composition of the invention.

The liquid mixture or mouthwash may include as excipients:

1-15% by weight of isomaltose and / or

1-4% by weight of glyceryl behenate and / or

1-15% by weight of limonene.

Isomaltose is a sweetener, while imonene is an ingredient that adds flavor and freshness. Glyceryl behenate is an anti-caking agent.

An additional object of the invention relates to a mouthwash or liquid mixture comprising the postbiotic composition described above.

When the composition, in accordance with the invention, is a soluble solid tablet, said tablet comprises:

0.2-40% by weight of the postbiotic composition of the invention and

60-99.8% by weight of at least one solid excipient.

The soluble solid tablet may dissolve in the subject's mouth.

In a particular embodiment, the soluble solid tablet of the invention comprises approximately 0.3% by weight of the postbiotic composition of the invention and approximately 99.7% by weight of at least one excipient, particularly approximately 1% by weight of the postbiotic composition of the invention and approximately 99% by weight of at least one excipient, particularly approximately 5% by weight of the postbiotic composition of the invention and approximately 95% by weight of at least an excipient, particularly approximately 10% by weight of the postbiotic composition of the invention and approximately 90% by weight of at least one excipient, particularly approximately 15% by weight of the postbiotic composition of the invention and approximately 85% by weight of at least one excipient, particularly approximately 20% by weight of the postbiotic composition of the invention and approximately 80% by weight of at least one excipient, particularly approximately 25% by weight of the postbiotic composition of the invention and approximately 75% by weight of at least one excipient, particularly approximately 30% by weight of the postbiotic composition of the invention and approximately 70% by weight of at least one excipient, particularly approximately 35% by weight of the postbiotic composition of the invention and approximately 65% by weight of at least one excipient, particularly approximately 40% by weight of the postbiotic composition of the invention and approximately 60% by weight of at least one excipient.

In another particular embodiment, the soluble solid tablet of the invention comprises between approximately 5-20% by weight of the postbiotic composition of the invention and approximately 95-80% by weight of at least one excipient.

In a particular embodiment, the soluble solid tablet can comprise the components of Table 2

Table 2: Example of soluble solid tablet composition

| Component | % by weight |
|---|---|
| silicon dioxide | 5 - 30 % |
| magnesium stearate | 5 - 10% |
| mint aroma | 1 - 10 % |
| guar gum | 2 - 50% |
| sorbitol | 3 - 10% |
| Composition of the invention | 0.2 - 40 % |

Guar gum is used because it is able to absorb water, forming a mixture that can thicken and adhere ingredients.

Sorbitol is used as a bulking agent to hold ingredients together.

Magnesium stearate is found as an additive in pills and tablets as a binder or emulsifier, and is used to contain the active ingredients together.

Silicon dioxide is used to protect tooth enamel.

An additional object of the invention relates to a soluble solid tablet comprising the postbiotic composition described above.

Other compositions of the tablets will be appropriate so long as these solid tablets are dissolved in the mouth of the person to whom they are administered.

The pharmaceutical compositions of the invention would be prepared following usual and known procedures in the prior art. A skilled person would know how to identify at what time of the preparation of toothpaste, mouthwash, bioadhesive gel and / or soluble solid tablet, the postbiotic composition of the invention should be added: for example, add the postbiotic composition of the invention to mouthwash, toothpaste, bioadhesive gel and / or soluble solid tablet and stir until the postbiotic composition is dissolved, or suspended.

Each of the terms "comprising", "consisting essentially of" and "consisting of' may be replaced by either of the other two terms. The term "a" may refer to one or a plurality of the elements they modify (for example, "a reactant" may mean one or more reactants), unless it is contextually clear that one or more of the elements is described. The term "approximately," as used herein, refers to a value within 10% of the underlying parameter (i.e., plus or minus 10%; for example, a weight of "approximately 100 grams" may include a weight between 90 grams and 110 grams). The use of the term "approximately" at the beginning of a list of values modifies each of the values (for example, "approximately 1, 2, and 3" refers to "approximately 1, approximately 2, and approximately 3"). When describing a list of values, the list includes all intermediate values and all fractional values thereof (for example, the list of values "80%, 85% or 90%" includes the intermediate value 86% and the fractional value 86.4%). When a listing of values is followed by the term "or more", the term "or more" is applied to each of the values listed (for example, the listing of "80%, 90%, 95% or more" refers to "80% or more, 90% or more, or 95% or more"). When describing a list of values, the listing includes all ranges between two of the listed values (for example, the listing of "80%, 90%, or 95%" includes ranges of "80% to 90%," "80% to 95%," and "90% to 95%"). See below for some examples of the application of the technology.

Additionally, the invention comprises the following clauses:

1. An orally administered postbiotic composition for use in the treatment and / or prevention of dysbiosis of the oral microbiota in a subject, comprising a mixture of lysates of probiotic microorganisms in an amount as a percentage by weight of the total weight of the lysates of microorganisms of the composition:

- 6% to 19% of bacterial lysates of the genus *Bacillus;*
- 4% to 8% of bacterial lysates of the genus *Bifidobacterium;*
- 15% to 25% of bacterial lysates of the genus *Lactobacillus;*
- 50% to 60% of yeast lysates of the genus *Saccharomyces;*
- 1.5% to 7% of bacterial lysates of the genus *Streptococcus.*

2. The composition, in accordance with the preceding clause, comprising lysates of probiotic microorganisms in an amount in percentage by weight with respect to the total weight of the lysates of microorganisms of the composition:

- 13% to 19% of bacterial lysates of the genus *Bacillus;*
- 4% to 8% of bacterial lysates of the genus *Bifidobacterium;*
- 15% to 25% of bacterial lysates of the genus *Lactobacillus;*

- 50% to 60% of yeast lysates of the genus *Saccharomyces;*
- 1.5% to 5% of bacterial lysates of the genus *Streptococcus.*

3. The composition, in accordance with any of clauses 1 and 2, wherein the percentage of *Lactobacillus* and *Streptococcus* lysates does not exceed 30% of the total weight.

4. The composition, in accordance with any of the preceding clauses, wherein the composition comprises between 1% and 99.5% by weight of lysates of microorganisms, particularly 5%-99% by weight of lysates of microorganisms, particularly 10%-96% by weight of lysates of microorganisms, 25%-95% by weight of lysates of microorganisms, or particularly 50%+10% by weight of lysates of microorganisms.

5. The composition, in accordance with any of the preceding clauses, wherein the bacterial lysates of the genus *Bacillus* are of the species that are selected from the group consisting of *Bacillus clausii, Bacillus coagulans, Bacillus licheniformis, Bacillus pumilus,* Bacillus *subtilis, Bacillus mesentericus, Bacillus paralicheniformes,* and combinations thereof.

6. The composition, in accordance with any of the preceding clauses, wherein the bacterial lysates of the genus *Bacillus* are of the species *Bacillus licheniformis, Bacillus pumilus* and Bacillus subtilis.

7. The composition, in accordance with any of the preceding clauses, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

8. The composition, in accordance with any of the preceding clauses, wherein the bacterial lysates of the genus *Bifidobacterium* are of the species *Bifidobacterium lactis* and *Bifidobacterium animalis subsp lactis.*

9. The composition, in accordance with any of the preceding clauses, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

10. The composition, in accordance with any of the preceding clauses, wherein the bacterial lysates of the genus *Lactobacillus* are of the species *Lactobacillus acidophilus, Lactobacillus casei, Lactobacillus fermentum, Lactobacillus paracasei, Lactobacillus plantarum, Lactobacillus reuteri, Lactobacillus rhamnosus,* and *Lactobacillus salivarius.*

11. The composition, in accordance with any of the preceding clauses, wherein the lysates of yeast genus *Saccharomyces* are of the species that are selected from the group consisting of *Saccharomyces cerevisiae, Saccharomyces boulardi,* and combinations thereof.

12. The composition, in accordance with any of the preceding clauses, wherein the lysates of yeast genus *Saccharomyces* are of the species *Saccharomyces cerevisiae.*

13. The composition, in accordance with any of the preceding clauses, wherein the bacterial lysates of the genus *Streptococcus* are of the species that are selected from the group consisting of *Streptococcus thermophilus, Streptococcus salivarius,* and combinations thereof.

14. The composition, in accordance with any of the preceding clauses, wherein the bacterial lysates of the genus *Streptococcus* are of the species *Streptococcus thermophilus.*

15. The composition, in accordance with any of the preceding clauses, wherein the bacterial lysates of the genus *Bacillus* are approximately 13% to 19% of the total lysates of the composition.

16. The composition, in accordance with any of the preceding clauses, wherein the percentage by weight of *Bacillus licheniformis, Bacillus pumilus* and *Bacillus subtilis* is approximately the same.

17. The composition, in accordance with any of the preceding clauses, wherein bacterial lysates of the genus *Bifidobacterium* are approximately 5% to 7% of the total lysates of the composition.

18. The composition, in accordance with any of the preceding clauses, where the percentage by weight of *Bifidobacterium lactis* is higher than the percentage by weight of *Bifidobacterium animalis subsp lactis.*

19. The composition, in accordance with any of the preceding clauses, wherein bacterial lysates of the genus *Lactobacillus* are about 20% to 24% of the total lysates of the composition.

20. The composition, in accordance with any of the preceding clauses, wherein the bacterial lysates of the genus *Lactobacillus* are *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius,* the percentage by weight of *Lactobacillus acidophilus* and *Lactobacillus casei* being different, the percentage by weight of *Lactobacillus reuteri* and *Lactobacillus rhamnosus* being different from the aforementioned but identical between themselves, the percentage by weight of *Lactobacillus paracasei* and *Lactobacillus salivarius* being different from the aforementioned but identical between themselves, the percentage by weight of *Lactobacillus fermentum* and *Lactobacillus plantarum* being different from the aforementioned but identical between themselves.

21. The composition, in accordance with any of the preceding clauses, wherein the bacterial lysates of the genus *Lactobacillus* are *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius,* the percentage by weight of *Lactobacillus casei* being higher than the rest of the *Lactobacillus* species.

22. The composition, in accordance with any of the preceding clauses, wherein the bacterial lysates of the genus *Lactobacillus* are *Lactobacillus acidophilus, Lactobacillus casei, Lactobacillus paracasei, Lactobacillus plantarum, Lactobacillus fermentum, Lactobacillus reuteri, Lactobacillus rhamnosus* and *Lactobacillus salivarius,* the percentage by weight of *Lactobacillus casei* being higher than that of *Lactobacillus acidophilus,* which in turn is higher than that of *Lactobacillus reuteri* and *Lactobacillus rhamnosus,* the percentage by weight of these 2 species being approximately identical, which in turn is superior to that of *Lactobacillus fermentum* and *Lactobacillus plantarum,* the percentage by weight of these 2 species being approximately identical, which in turn is higher than that of *Lactobacillus paracasei* and *Lactobacillus salivarius,* the percentage by weight of these 2 species being approximately identical.

23. The composition, in accordance with any of the preceding clauses, wherein the lysates of yeast of the genus *Saccharomyces* are approximately between 52% to 57% of the total lysates of the composition.

24. The composition, in accordance with any of the preceding clauses, wherein the lysates of yeast of the genus *Streptococcus* are approximately between 3% to 4% of the total lysates of the composition.

25. The composition, in accordance with any of the preceding clauses, wherein the composition comprises lysates of probiotic microorganisms in an amount in percentage by weight of the total lysates of the composition according to composition A.

26. The composition, in accordance with any of the preceding clauses, wherein the composition comprises lysates of probiotic microorganisms in an amount in percentage by weight of the total lysates of the composition according to composition B.

27. The composition, in accordance with any of the preceding clauses 1 to 24, wherein the composition comprises lysates of probiotic microorganisms in an amount in percentage by weight of the total lysates of the composition according to composition C.

28. The composition, in accordance with any of the preceding clauses 1 to 24 and 27, wherein the composition comprises lysates of probiotic microorganisms in an amount in percentage by weight of the total lysates of the composition according to composition D.

29. The composition, in accordance with any of the preceding clauses 1 to 24 and 28, wherein the composition comprises lysates of probiotic microorganisms in an amount in percentage by weight of the total lysates of the composition according to composition E.

30. The composition, in accordance with any of the preceding clauses 1 to 24 and 29, wherein the composition comprises lysates of probiotic microorganisms in an amount in percentage by weight of the total lysates of the composition according to composition F.

31. The composition, in accordance with any of the preceding clauses 1 to 24 and 25 and 27 wherein the composition comprises lysates of probiotic microorganisms in an amount in percentage by weight of the total lysates of the composition according to composition G.

32. The composition, in accordance with any of the preceding clauses 1 to 24 and 25 and 27 to 29 and 31, wherein the composition comprises lysates of probiotic microorganisms in an amount in percentage by weight of the total lysates of the composition according to composition H.

33. The composition, in accordance with any of the preceding clauses 1 to 24 and 25 and 27 to 29 and 31 to 32, wherein the composition comprises lysates of probiotic microorganisms in an amount in percentage by weight of the total lysates of the composition according to composition I.

33. The composition, in accordance with any of the preceding clauses 1 to 24 and 25 and 27 to 29 and 31 to 33, wherein the composition comprises lysates of probiotic microorganisms in an amount in percentage by weight of the total lysates of the composition according to composition J.

34. The composition, in accordance with any of the preceding clauses, in which the percentage by total weight of bacterial lysates of *Lactobacillus* and *Streptococcus* species is equal to or less than about 30%.

35. The composition, in accordance with any of the preceding clauses, wherein the dysbiosis of the oral microbiota comprises one or more of the following conditions: dental caries, gingivitis, periodontitis and halitosis.

36. A procedure for obtaining a postbiotic composition of oral administration for use in the treatment and / or prevention of oral microbiota dysbiosis, according to any of the clauses 1 to 35 comprising:

- Cultivating the selected microbial species

- Filter or centrifuge the cultures until an adequate biomass of the selected microorganisms is obtained

- Lysis through cycles of freezing and thawing, and sonication of the obtained the biomasses

37. The process, in accordance with the preceding clause, comprising drying by atomization or lyophilization of the biomasses of bacterial lysates obtaining a product in the form of dry powder.

38. A pharmaceutical composition comprising an effective pharmaceutical quantity of the postbiotic composition, in accordance with any of the clauses 1 to 35, and a pharmaceutically acceptable excipient.

39. The pharmaceutical composition, in accordance with the preceding clause, which is presented in the form of toothpaste, mouthwash, bioadhesive gel or soluble solid tablets.

40. The pharmaceutical composition, in accordance with the preceding clause, which is a toothpaste comprising:

0.2-40% by weight of the postbiotic composition defined in any of clauses 1 to 35 and

99.8-60% by weight of at least one excipient.

41. The pharmaceutical composition, in accordance with the preceding clause, wherein the excipient is selected from preservatives, flavorings, fragrances, binders, foaming agents, humectants, abrasives, and combinations thereof.

42. The pharmaceutical composition, in accordance with the preceding clause, comprising 20-60% by weight of the humectant.

43. The pharmaceutical composition, in accordance with any of the clauses 41 to 42, comprising 30-99.8% by weight of the abrasive.

44. The pharmaceutical composition, in accordance with any of clauses 40 to 43, comprising the following excipients: sodium benzoate, sodium saccharin, lemon oil, cellulose gum, sodium lauryl sulphate, glycerin, sorbitol, dicalciumphosphate, and hydrated dental silica.

45. A toothpaste comprising the postbiotic composition, in accordance with any of the clauses 1 to 35.

46. A toothpaste, in accordance with the preceding clause, comprising:

    0.2-40% by weight of the postbiotic composition defined in any of clauses 1 to 35 and

    99.8-60% by weight of at least one excipient.

47. A toothpaste, in accordance with the preceding clause, wherein the excipient is selected from one or more of clauses 41 to 44.

48. The pharmaceutical composition, in accordance with clause 39, which is a mouthwash comprising:

    0.2-40% by weight of the postbiotic composition defined in any of clauses 1 to 35 and

    1-40% by weight of at least one excipient.

49. The pharmaceutical composition, in accordance with the preceding clause, wherein the excipient of the mouthwash is selected from sweeteners, flavorings, anti-caking agents, and combinations thereof.

50. A mouthwash comprising the postbiotic composition, in accordance with any of the clauses 1 to 35.

51. A mouthwash, in accordance with the preceding clause, comprising:

    0.2-40% by weight of the postbiotic composition defined in any of clauses 1 to 35 and

    1-40% by weight of at least one excipient.

52. A mouthwash, in accordance with the preceding clause, wherein the excipient is selected from one or more of those in clause 49.

53. The pharmaceutical composition, in accordance with clause 39, which is a soluble solid tablet comprising:

    0.2-40% by weight of the composition defined in any of clauses 1 to 35 and

    99.8-60% by weight of at least one solid excipient.

54. The pharmaceutical composition, in accordance with the preceding clause, wherein the excipients are silicon dioxide, magnesium stearate, mint flavoring, guar gum and sorbitol.

55. A soluble solid tablet comprising the postbiotic composition, in accordance with any of the clauses 1 to 35.

56. The soluble solid tablet, in accordance with the preceding clause, comprising:

    0.2-40% by weight of the postbiotic composition defined in any of clauses 1 to 35 and

    99.8-60% by weight of at least one solid excipient.

57. The soluble solid tablet, in accordance with the preceding clause, wherein the excipient is selected from one or more of those in clause 54.

58. The pharmaceutical composition, in accordance with clause 39, which is a bioadhesive gel comprising:

    0.2-40% by weight of the postbiotic composition defined in any of clauses 1 to 35 and

99.8-60% by weight of at least one excipient.

59. The pharmaceutical composition, in accordance with the preceding clause, wherein the excipients of the bioadhesive gel are Hyaluronic acid at 0.2% by weight approximately, Chlorhexidine at 0.2% by weight approximately, and Panthenol at 5% by weight approximately.

60. A bioadhesive gel comprising the postbiotic composition, in accordance with any of the clauses 1 to 35.

61. The soluble solid bioadhesive gel, in accordance with the preceding clause, comprising:

0.2-40% by weight of the postbiotic composition defined in any of clauses 1 to 35 and

99.8-60% by weight of at least one excipient.

62. The soluble solid tablet, in accordance with the preceding clause, wherein the excipient is selected from one or more of those in clause 59.

## BRIEF DESCRIPTION OF THE FIGURES

To complement the description and enable a better understanding of the invention, we are including as an integral part of said description, a set of figures where, for illustrative and nonlimiting purposes, graphs of the experimental results obtained in the examples contained in this document are presented.

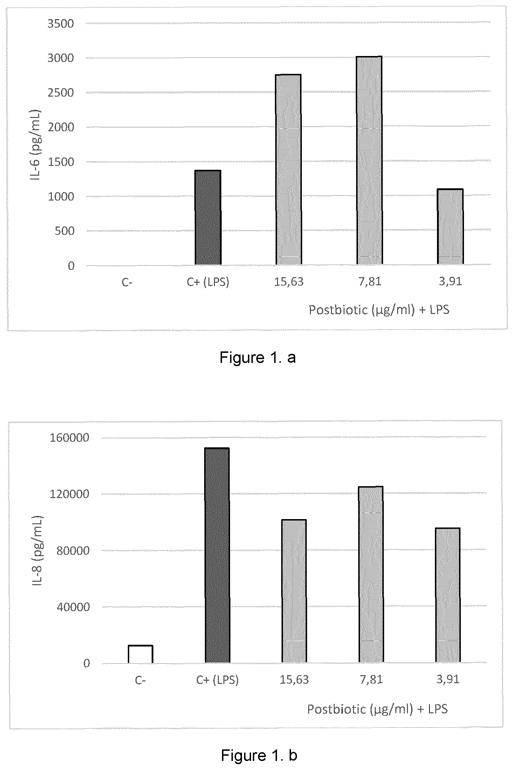

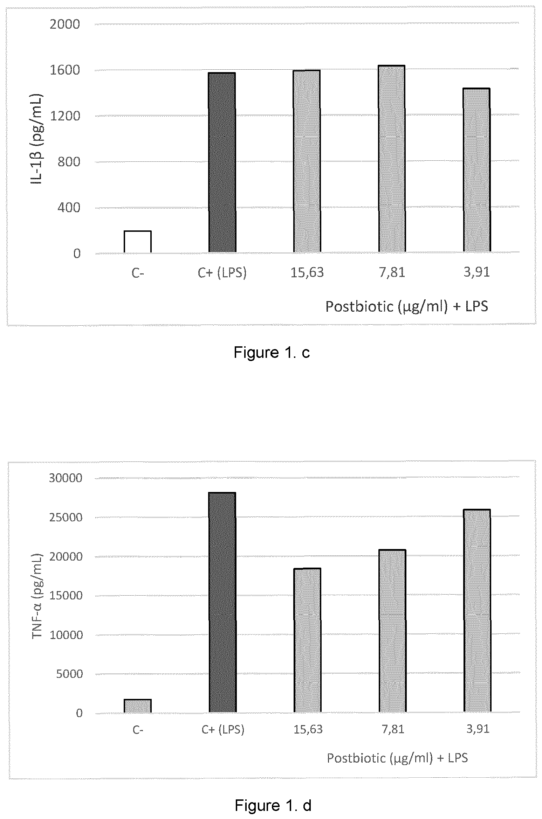

**Figure 1.** Protein secretion IL-6 (Figure 1.a), IL-8 (Figure 1.b), IL-1β (Figure 1.c) and TNF-α (Figure 1.d) by THP-1 cells stimulated with LPS under different concentrations of the postbiotic composition of the invention. The bars represent the average of three replicas and the error bars correspond to the standard deviation. Negative control (C-) represents a condition in which cells were not exposed to any compound. Positive control (C+) represents a condition in which cells were exposed to an inflammatory stimulus of LPS.

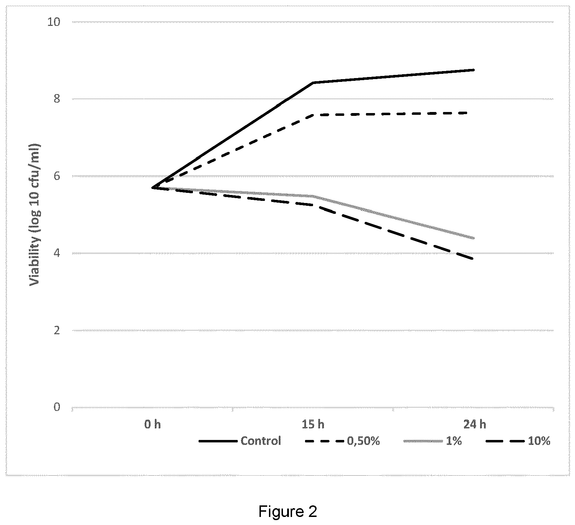

**Figure 2.** Viability of *Streptococcus mutans* in the presence of the postbiotic composition of the invention.

## MODES OF EMBODIMENT OF THE INVENTION

### EXAMPLE 1. IMMUNOMODULATORY ACTIVITY OF THE COMPOSITION OF THE INVENTION IN CELLS THP-1

Objective

Periodontitis is a common inflammatory periodontal disease that affects a large portion of the population worldwide. The causative bacteria release chemicals that activate the innate immune system to release pro-inflammatory cytokine substances that contribute to further progression. This activates the acquired immune system, which leads to further progression of the disease. As the immune response progresses, immune mediators are released that cause soft tissue inflammation and bone damage. There is a large growth of proinflammatory cytokines.

The objective of this study is to examine the immunomodulatory activity in vitro that a postbiotic, formed by a mixture of lysates of microorganisms, can have on the regulation of cytokine secretion in THP-1 cells. To this end, the influence on the expression of the main proinflammatory cytokines in macrophages is analyzed.

**Materials and Methods:**

The lysate of microorganisms was obtained from a mixture of lysates of strains of *Lactobacillus sp., Bacillus sp., Bifidobacterium sp., Saccharomyces sp.* and *Streptococcus sp.*

The composition consisted of lysates of *Bacillus subtilis* (5%), *Bacillus licheniformis* (5%), *Bacillus pumilus* (5%), *Bifidobacterium animalis subsp lactis* (1%), *Bifidobacterium lactis* (5%), *Lactobacillus acidophilus* (4%), *Lactobacillus casei* (5%), *Lactobacillus fermentum* (2%), *Lactobacillus paracasei* (1%), *Lactobacillus plantarum* (2%), *Lactobacillus reuteri* (3%), *Lactobacillus rhamnosus* (3%), *Lactobacillus salivarius* (1%), *Saccharomyces cerevisiae* (55%) and *Streptococcus thermophilus* (3%), in an amount as a percentage by weight of the total weight of the lysates of the composition.

Each of them was grown under standard culture conditions specific to each species: *Bacillus* in Nutrient broth at 30°C for 24h, *Bifidobacterium animalis* in MRS at 37°C for 48h in anaerobic atmosphere, *Lactobacillus sp.* in MRS at 37°C for 24h, *Streptococcus thermophilus* in BHI at 37°C for 24h, and *Saccharomyces cerevisiae* in YEPD at 26°C. Once grown, the cultures were centrifuged, separating the biomass by lysing it by sonication. The lysate was lyophilized to obtain the powder that was subsequently added in the different tests.

Macrophages were obtained from the THP-1 cell line (human monocyte cell line, ATCC® TIB-202). These cells were stored in Gaiker's culture bank and checked for the absence of contaminating mycoplasmas immediately after thawing. The cells were grown in suspension at 37°C in a humid atmosphere of 5% CO2. They were kept in culture medium RPMI + 10 % FBSi + 50 $\mu$M $\beta$-mercaptoethanol, for a minimum of two weeks before any experiment. The cells were subcultured before reaching 80% confluence (cell concentration / volume).

A cell suspension of $1\,10^5$ cells/mL was prepared 24 hours before the immunomodulatory activity test. $1\,10^4$ cells / well were dispensed in 96-well plates in the presence of 0.31 $\mu$g/mL of PMA (phorbol-12-myristate-13-acetate) to differentiate monocytes from macrophages. The plates were incubated at 37°C and 5% $CO_2$ for 24 hours.

For the immunomodulation assay, THP-1 PMA-differentiated cells with different concentrations of the composition of the invention were incubated for 24h in the presence of an inflammatory stimulus, lipopolysaccharide, LPS (20ug/ml). Non-inflamed cells (cells exposed to unstimulated culture medium) were used as a negative control. The supernatant was collected at 24h. Levels of cytokines IL-8, TNF-$\alpha$, IL-6 and IL-1$\beta$ were quantified using an ELISA detection kit in accordance with the manufacturer's instructions (Human IL-8 Elisa Kit. Invitrogen: KHC0082; Human IL-6 Elisa Kit. Invitrogen: KHC0062; Human IL-1$\beta$ Elisa Kit. Invitrogen: KHC0012; Human TNF-alpha Elisa Kit. Invitrogen; 10008932).

The study of the cytotoxicity of the products was performed with a feasibility test by MTT. MTT (a yellow tetrazolium) is reduced to purple formazan in living cells, evaluating cellular metabolic activity and, therefore, cell viability. Cells grown in 96-well plates were treated with 8 concentrations of the products under study for 24 hours (37°C, 5% $CO_2$). In parallel, 8 concentrations of a positive control (sodium dodecyl sulfate (SDS)) were also tested to validate the assay. After 24 hours of incubation with the postbiotic or positive control, the cells were washed with PBS and stained with a solution of MTT incubating at 37°C for 2 hours. At the end of this incubation period, the staining medium was removed and 100 $\mu$l of DMSO / well was added to solubilize the colored precipitate. The absorbance was read at 540 nm in the spectrophotometer plate reader. The percentage of cell viability was calculated in relation to the negative control, a condition in which the cells were not exposed to any product.

$$\% \text{ cell viability} = AbT/AbC \times 100$$

AbT = absorbance at 540 nm after 72 hours of treatment

AbC = absorbance at 540 nm after 72 hours without treatment (negative control)

**RESULTS**

Mycoplasma test

The results obtained in the mycoplasma test are shown in Table 3.

Table 3. Ratio of mycoalert obtained in each cell line

| Ratio of mycoalert obtained in each cell line | | | |
|---|---|---|---|
| Cell | Reading A | Reading B | Ratio (reading B/reading A) |
| THP-1 | 3503 | 1265 | 0.361 |

The ratios obtained in the MycoAlert mycoplasma® detection kit (Lonza LT07-318) were less than 0.9, indicating the absence of mycoplasma contamination.

Cytotoxicity test

The cytotoxic effect of the compound was tested in vitro, by MTT assay, in the THP-1 cell line. For this purpose, eight concentrations of the composition of the invention were analyzed: 500 $\mu$g/mL, 250 $\mu$g/mL, 125 $\mu$g/mL, 62.5 $\mu$g/mL, 31.25 $\mu$g/mL, 15.63 $\mu$g/mL, 7.81 $\mu$g/mL, and 3.91 $\mu$g/mL. In parallel, different concentrations of sodium dodecyl sulfate (SDS) were used as a positive control (not shown). Table 4

Table 4: Percentage of cell viability

| | C- | Composition of the invention (µg/mL) | | | | | | | | C+ (LPS ) |
|---|---|---|---|---|---|---|---|---|---|---|
| | | 500 | 250 | 125 | 62,5 | 31,25 | 15,63 | 7,81 | 3,91 | |
| Replica | 103,431 | 71,852 | 78,882 | 84,161 | 88,152 | 94,615 | 101,499 | 101,602 | 104,057 | 105,928 |
| | 99,722 | 70,977 | 79,723 | 86,616 | 90,967 | 93,877 | 97,997 | 100,641 | 103,388 | 102,684 |
| | 104,735 | 71,972 | 79,946 | 85,002 | 88,547 | 95,68 | 98,529 | 98,959 | 100,435 | 102,993 |
| | 100,255 | 68,367 | 77,097 | 84,161 | 89,972 | 93,791 | 97,122 | 98,22 | 104,727 | 99,662 |
| | 95,002 | 66,822 | 74,83 | 80,693 | 85,749 | 89,877 | 95,422 | 97,551 | 95,199 | 95,628 |
| | 96,856 | 65,226 | 76,822 | 82,272 | 88,409 | 89,328 | 93,671 | 97,688 | 99,027 | 100,177 |
| | C- | Composition of the invention (µg/mL) | | | | | | | | C+ (LPS) |
| | | 500 | 250 | 125 | 62,5 | 31,25 | 15,63 | 7,81 | 3,91 | |
| Average | 100 | 69,203 | 77,883 | 83,817 | 88,633 | 92,861 | 97,373 | 99,11 | 101,139 | 101,179 |
| Desvest | 3,811 | 2,829 | 1,985 | 2,079 | 1,78 | 2,619 | 2,695 | 1,663 | 3,652 | 3,528 |

Table 4 shows the percentage of cell viability after 24 hours of exposure to different concentrations of the compound under study by MTT assay in THP-1 cell lines differentiated with PMA. Negative control (C-) represents a condition in which cells were not exposed to any compound.

The results showed that concentrations 15.63 µg/mL, 7.81 µg/mL and 3.91 µg/mL did not alter the viability of THP1 cells and were selected for the immunomodulation assay.

Immunomodulatory activity

THP-1 cells differentiated from macrophages were exposed simultaneously to an inflammatory stimulus with LPS and different concentrations of the composition of the invention according to the results of cytotoxicity, for 24h. After exposure time, supernatants were collected and analyzed. The immunomodulatory effect was analyzed by quantifying the secreted levels of IL-6, IL-8, IL-1β and TNF-α. The results obtained are represented in Figure1.

All concentrations tested reduce the secretion of IL-8 and TNF-α in inflamed cells. It was observed that TNF-α levels decrease with increasing concentration, while decreasing the concentration increased the levels to close to those of cells inflamed with LPS without the postbiotic. In the case of IL-8, the levels were in any case lower with respect to the absence of postbiotics. Only at low concentrations is a slight decrease observed.

IL-6 secretion was also analyzed, the results of which are summarized in Figure 1. In this case, the levels were higher than those of macrophages treated with LPS alone. This tendency is different from when the concentration of the composition of the invention decreases, such that the level of interleukin is lower than that of the positive control.

**CONCLUSION**

The study shows that the composition of the invention under the conditions described presents an immunomodulatory activity in all concentrations tested (15.63 μg/mL, 7.81 μg/mL and 3.91 μg/mL). LPS stimulation increases the production of IL-6, IL-8, TNF-α and IL-1β in THP-1 cells. When inflamed cells are also treated with the postbiotic the secreted levels of IL-8 and TNF-α compared to cells treated with LPS alone decrease. On the other hand, the response of the cells having been also treated with postbiotic increasing the secretion of IL-6 and IL-1β, remained practically unchanged with respect to only LPS.

In periodontitis, the levels of these cytokines usually have elevated levels. The immunomodulatory activity of the postbiotic could decrease the levels of TNF-α in favor of IL-6 by its inhibitory action at high concentrations, while this tendency would be the opposite at low concentrations. It would also decrease the secretion of IL-8, induced mainly by periodontopathogens such as *Fusobacterium nucleatum* and *Porphyromonas gingivalis.*

**EXAMPLE 2. EVALUATION OF THE REGULATION OF THE ORAL MICROBIOTA ASSOCIATED WITH DENTAL CARIES**

Objective

One of the consequences of dysbiosis of the oral microbiota is the enrichment in certain aciduric bacterial species (especially *Streptococcus* and *Lactobacillus*) that change the microenvironment in which they are found to a more acidic one, which inhibits beneficial organisms that preferably grow at neutral pH. Among the species that are most favored by this enrichment is S. *mutans,* which is characterized by its cariogenic properties and is considered one of the main pathogens associated with caries (Ojeda-Garcés, Juan Carlos, Oviedo-Garcia, Eliana, & Salas, Luis Andrés. (2013). Streptococcus mutans and dental caries. CES Dentistry, 26(1), 44-56). On the other hand, this bacterium also controls the overgrowth of others that can be harmful to oral health, so it is necessary to find a balance between them.

The objective of the study is to modulate the growth of *Streptococcus mutans* and therefore its effect on the development of dental caries, with a postbiotic formulation that would also act against other oral diseases, such that it would have a general impact on oral health. To this end, cultures of *S. mutants CECT 479* will be carried out and the effect of different species of bacteria and yeasts will be analyzed, as well as their mixture at 24h of growth to know how this affects the viability of the bacteria.

Method

A culture of *Streptococcus mutans* CECT 479 is prepared in a flask containing 200 ml of sterile Oxoid BHI (Brain Health Infusion Broth) in accordance with the supplier's instructions (https://www.cect.org/vstrn.php?lan=en&cect=479). In the laminar flow chamber, 700 ul of a bacterium vial is inoculated into the flask under sterile conditions. It is incubated in the orbital agitator at 200 rpm at 37°C. After 24h, 1 ml of well-homogenized bacterial suspension is taken in 9 ml of peptone water (Merck), and serial dilutions are made in peptone water, seeding them in BHA (Brain Heart Agar) plates of Oxoid. The viability at 24h of growth is $1.8 \times 10^8$ cfu/ml.

The postbiotic composition of the invention consisted of lysates of *Bacillus subtilis* (5%), *Bacillus licheniformis* (5%), *Bacillus pumilus* (5%), *Bifidobacterium animalis subsp lactis* (1%), *Bifidobacterium lactis* (5%), *Lactobacillus acidophilus* (4%), *Lactobacillus casei* (5%), *Lactobacillus fermentum* (2%), *Lactobacillus paracasei* (1%), *Lactobacillus plantarum* (2%), *Lactobacillus reuteri* (3%), *Lactobacillus rhamnosus* (3%), *Lactobacillus salivarius* (1%), *Saccharomyces cerevisiae* (55%) and *Streptococcus thermophilus* (3%), in an amount as a percentage by weight of the total weight of the lysates of the composition.

Each of them was grown under the normal culture conditions of each species: *Bacillus* in Nutrient broth at 30°C for 24h, *Bifidobacterium* in MRS at 37°C for 48h in anaerobic atmosphere, *Lactobacillus sp.* in MRS at 37°C for 24h, *Streptococcus thermophilus* in BHI at 37°C for 24h and *Saccharomyces cerevisiae* in YEPD at 26°C. Once grown, the cultures were centrifuged, separating the biomass by lysing it by sonication The lysate was lyophilized to obtain the powder that was subsequently added in the different tests.

The postbiotic composition of the invention is resuspended in BHI in different percentages in three flasks, passing it through a sterile filter of 0.4 microns. The final percentages are 0%, 0.5%, 1% and 10% in a final volume of 100 ml of BHI. The flasks are inoculated with 100 ul of the preinoculum of *S. mutans* and incubated in an orbital stirrer at 37° C, with an agitation of 200 rpm for 24h. After this time, 1ml of well-homogenized bacterial suspension is taken in 9 ml of peptone water (Merck). Serial dilutions are made and placed in BHA plates, incubating them for 24h at 37°C.

Results

The results indicate that the postbiotic composition of the invention affects the growth of *S. mutans,* slowing the growth rate. As the percentage of postbiotic increases, the growth of *S. mutans* slows down, with greater inhibition with increasing concentration.

The results shown in Figure 2 indicate that after 24h of incubation the control of *S. mutans* came to have a viability greater than $10^8$ cfu/ml. In the presence of the composition of the invention, the growth of the bacterium was lower than in the control without postbiotic. As the concentration of the composition increased, the growth of *Streptococcus mutans* decreased in all cases, reaching a viability inferior than the control at 24h.

**CONCLUSION**

In the presence of the composition, the growth of the bacterium was lower than in the control without postbiotic, this being more evident when increasing the presence of the same.

These results indicate that the use of the composition of the invention for dental hygiene on a regular basis can modulate the growth of the pathogen without eliminating it from the oral ecosystem. Therefore, its use can modulate the oral microbiota by inhibiting the overgrowth of one of the bacteria responsible for the formation of dental caries.

**EXAMPLE 3. MODULATION OF THE ORAL MICROBIOTA BY INHIBITING BACTERIA INVOLVED IN THE DEVELOPMENT OF PERIODONTITIS**

The oral microbial ecosystem plays an essential role in maintaining human health. Its diversity may decrease with age and its alteration may be intimately associated with both oral and systemic diseases. The presence of some pathogens can cause alterations in the environment and alter the proportion of other beneficial microorganisms in the niches. It is therefore crucial to improve protection against these and thus maintain the dynamic balance of the oral microbiota.

To determine whether the postbiotic composition of the invention was capable of producing modulation of the oral microbiota, the same composition was used as in Example 2, formed by lysates of different species of *Lactobacillus sp., Bacillus sp., Bifidobacterium sp., Streptococcus sp.* and *Saccharomyces sp.* This postbiotic was tested in a planktonic culture with species involved in the development of periodontitis, such as *A. actinomycetemcomitans, P. intermedia,* and *P.gingivalis.* As a result, at 24h and 48h, using two concentrations, a reduction in plaque counts of the levels of periodontal pathogens was observed.

These results demonstrate that the postbiotic composition of oral administration is able to modulate the oral microbiota by reducing pathogenic microorganisms.

**EXAMPLE 4. CLINICAL STUDY**

- Clinical study in periodontal patient with an N of 35 per arm. Two branches, double blind.

- **Product presentation.** Bioadhesive gel

- **Dosage:** three administrations per day.

- **Application time:** 6 months.

- **Variables to study:** 1) Periodontal clinical variables, 2) Microbiological variables at the active site, 3) Markers of inflammation.

**Objective:**

To analyze whether the postbiotic composition of the invention can re-balance the composition and physiological function of the microbiota of periodontal patients, and if this modification is accompanied by periodontal clinical improvement: loss of oral inflammation, total or partial decrease in gum bleeding, and decrease in pathogens, among others, as well as variation of inflammation markers: IL-6, IL-8, IL-1β.

**Study Design:**

Observational study of a series of 70 patients in the early phase of the periodontal disease gingivitis. 35 patients used the bioadhesive gel Perio·aid® comprising the postbiotic composition of the invention (active), and another 35 affected patients used a bioadhesive gel Perio·aid® without the postbiotic composition (placebo).

Patients applied the bioadhesive gel three times a day to their gums for an estimated time of 3-5 minutes. Later it was removed with mouthwash. Samples were taken for analysis at the beginning of the trial (time = 0), at 3 months (time = 1), and at 6 months (time = 2), which was the final time established. Sampling was performed by a specialist.

The inclusion criterion was patients of both sexes, older than 18 years of age. All patients were administered the bioadhesive gel three times a day for at least 24 weeks. The bioadhesive gel (active) comprises a dose of the postbiotic composition of the invention of 400 mg, that is, 1200 mg daily, (3 daily doses). The clinical periodontal improvement and variation of the markers of inflammation were measured. Before starting treatment and then weekly, all the patients had to fill out a questionnaire and it was handed in at subsequent visits. The composition of the invention was administered in the form of a gel or toothpaste.

**Results:**

The results of the clinical trial show that treatment with the composition of the invention reduces periodontal diseases and that there is an improvement in the oral health of most patients.

**Claims**

1. An orally administered postbiotic composition for use in the treatment and / or prevention of dysbiosis of the oral microbiota in a subject, comprising a mixture of lysates of probiotic microorganisms in an amount as a percentage by weight of the total weight of the lysates of microorganisms of the composition:

   - 6% to 19% of bacterial lysates of the genus *Bacillus;*
   - 4% to 8% of bacterial lysates of the genus *Bifidobacterium;*
   - 15% to 25% of bacterial lysates of the genus *Lactobacillus;*
   - 50% to 60% of yeast lysates of the genus *Saccharomyces;*
   - 1.5% to 7% of bacterial lysates of the genus *Streptococcus.*

2. The composition, in accordance with the preceding claim, wherein the bacterial lysates of the genus *Bacillus* are of the species that are selected from the group consisting of *Bacillus clausii, Bacillus coagulans, Bacillus licheniformis, Bacillus pumilus, Bacillus subtilis, Bacillus mesentericus Bacillus paralicheniformes,* and combinations thereof.

3. The composition, in accordance with either of the preceding claims, wherein the bacterial lysates of the genus *Bifidobacterium* are of the species *Bifidobacterium animalis subsp lactis, Bifidobacterium bifidum, Bifidobacterium breve, Bifidobacterium lactis, Bifidobacterium longum, Bifidobacterium infantis, Bifidobacterium animalis,* and combinations thereof.

4. The composition, in accordance with any of the preceding claims, wherein the bacterial lysates of the genus *Lactobacillus* are of the species that are selected from the group consisting of *Lactobacillus lactis, Lactobacillus acidophilus, Lactobacillus bulgaricus, Lactobacillus casei, Lactobacillus fermentum, Lactobacillus paracasei, Lactobacillus plantarum, Lactobacillus reuteri, Lactobacillus rhamnosus Lactobacillus salivarius, Lactobacillus helvéticus, Lactobacillus gasseri, Lactobacillus johnsonii, Lactobacillus brevis, Lactobacillus kefiri,* and combinations thereof.

5. The composition, in accordance with any of the preceding claims, wherein the lysates of yeast genus *Saccharomyces* are of the species that are selected from the group consisting of *Saccharomyces cerevisiae, Saccharomyces boulardi,* and combinations thereof.

6. The composition, in accordance with any of the preceding claims, wherein the bacterial lysates of the genus *Streptococcus* are of the species that are selected from the group consisting of *Streptococcus thermophilus, Streptococcus salivarius,* and combinations thereof.

7. The composition, in accordance with any of the preceding claims, wherein the percentage by weight of *Bacillus licheniformis, Bacillus pumilus* and *Bacillus subtilis* is approximately the same.

8. The composition, in accordance with any of the preceding claims, wherein the percentage by weight of *Bifidobacterium lactis* is greater than the percentage by weight of *Bifidobacterium animalis subsp lactis.*

9. The composition, in accordance with any of the preceding claims, wherein the bacterial lysates of the genus *Lactobacillus* are of the species *Lactobacillus acidophilus, Lactobacillus casei, Lactobacillus paracasei, Lactobacillus plantarum, Lactobacillus fermentum, Lactobacillus reuteri, Lactobacillus rhamnosus,* and *Lactobacillus salivarius,*

   - the percentage by weight of *Lactobacillus acidophilus* and *Lactobacillus casei* being different between themselves,
   - the percentage by weight of *Lactobacillus reuteri* and *Lactobacillus rhamnosus* being lower than that of the two aforementioned species but identical between themselves,
   - the percentage by weight of *Lactobacillus fermentum* and *Lactobacillus plantarum* being lower than that of the four aforementioned species but identical between themselves, and
   - the percentage by weight of *Lactobacillus paracasei* and *Lactobacillus salivarius* being different from that of the six aforementioned species but identical between themselves.

10. The composition, in accordance with any of the preceding claims, wherein the composition comprises lysates of probiotic microorganisms in an amount in percentage by weight of the total lysates of the composition:

    - 6% to 19% of bacterial lysates of *Bacillus licheniformis, Bacillus pumilus* and *Bacillus subtilis;*
    - 4% to 8% of bacterial lysates of *Bifidobacterium animalis subsp lactis* and *Bifidobacterium lactis;*
    - 15% to 25% of bacterial lysates of *Lactobacillus acidophilus, Lactobacillus casei, Lactobacillus fermentum, Lactobacillus paracasei, Lactobacillus plantarum, Lactobacillus reuteri, Lactobacillus rhamnosus,* and *Lactobacillus salivarius;*
    - 50% to 60% of yeast lysates of *Saccharomyces cerevisiae;*
    - 1.5% to 7% of bacterial lysates of *Streptococcus thermophilus.*

11. The composition, in accordance with any of the preceding claims, wherein the composition comprises lysates of probiotic microorganisms in an amount in percentage by weight of the total lysates of the composition:

    - 6% to 19% of bacterial lysates of the species *Bacillus licheniformis, Bacillus pumilus* and *Bacillus subtilis,* the percentage by weight of *Bacillus licheniformis, Bacillus pumilus* and *Bacillus subtilis* being approximately the same;
    - 4% to 8% of bacterial lysates of the species *Bifidobacterium animalis subsp lactis* and *Bifidobacterium lactis,* the percentage by weight of *Bifidobacterium lactis* being higher than the percentage by weight of *Bifidobacterium animalis subsp lactis;*
    - 15% to 25% of bacterial lysates of the species *Lactobacillus acidophilus, Lactobacillus casei, Lactobacillus paracasei, Lactobacillus plantarum, Lactobacillus fermentum, Lactobacillus reuteri, Lactobacillus rhamnosus,* and *Lactobacillus salivarius,* the percentage by weight of *Lactobacillus casei* being higher than that of *Lactobacillus acidophilus,* the percentage by weight of *Lactobacillus reuteri* and *Lactobacillus rhamnosus* being lower than that of *L. casei* and *L. acidophilus* but the same between themselves, the percentage by weight of *Lactobacillus paracasei* and *Lactobacillus salivarius* being lower than that of the four aforementioned species but the same between themselves, the percentage by weight of *Lactobacillus fermentum* and *Lactobacillus plantarum* being lower than that of the six aforementioned species but the same between themselves;
    - 50% to 60% of yeast lysates of *Saccharomyces cerevisiae;*
    - 1.5% to 7% of bacterial lysates of *Streptococcus thermophilus.*

12. The composition, in accordance with any of the preceding claims, wherein the dysbiosis of the oral microbiota comprises one or more of the following conditions: dental caries, gingivitis, periodontitis and halitosis.

13. The composition, in accordance with the preceding claim, wherein dental caries are caused, at least in part, by *S. mutans*.

14. A pharmaceutical composition comprising an effective pharmaceutical amount of the composition, in accordance with any of claims 1 to 13, and a pharmaceutically acceptable excipient, for use in the treatment and / or prevention of dysbiosis of the oral microbiota in a subject.

15. The pharmaceutical composition, in accordance with the preceding claim, which is presented in the form of toothpaste, mouthwash, bioadhesive gel or soluble solid tablet.

Figure 1. a

Figure 1. b

Figure 1. c

Figure 1. d

Figure 2

Europäisches
Patentamt

European
Patent Office

Office européen
des brevets

# EUROPEAN SEARCH REPORT

**Application Number**

EP 23 38 2662

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ES 2 783 885 A1 (IGEN BIOLAB GROUP AG [CH]) 18 September 2020 (2020-09-18) * the whole document * | 1-15 | INV. A61K35/74 A23L33/135 A61K9/00 A61K35/742 A61K35/745 A61K35/747 A61P1/02 A61Q11/00 |
| Y | US 2022/273735 A1 (HO HSIEH-HSUN [TW] ET AL) 1 September 2022 (2022-09-01) * see claims, [041], Table 3, examples * | 1-15 | |
| Y | BASIR LEILA ET AL: "Effect of postbiotic-toothpaste on salivary levels of IgA in 6- to 12-year-old children: Study protocol for a randomized triple-blind placebo-controlled trial", FRONTIERS IN PEDIATRICS, vol. 10, 12 December 2022 (2022-12-12), XP093139294, CH ISSN: 2296-2360, DOI: 10.3389/fped.2022.1042973 * see Abstract and Table 1 * | 1-15 | |
| Y | MORAES RENATA MENDONÇA ET AL: "Outside the limits of bacterial viability: Postbiotics in the management of periodontitis", BIOCHEMICAL PHARMACOLOGY, ELSEVIER, US, vol. 201, 2 May 2022 (2022-05-02), XP087092985, ISSN: 0006-2952, DOI: 10.1016/J.BCP.2022.115072 [retrieved on 2022-05-02] * see abstract and Table 1 * | 1-15 | **TECHNICAL FIELDS SEARCHED (IPC)**<br><br>A61K<br>A23L<br>A61P<br>A61Q |
| Y | WO 2019/125204 A1 (OBSHCHESTVO S OGRANICHENNOY OTVETSTVENNOSTYU SPLAT KOSMETIKA [RU]) 27 June 2019 (2019-06-27) * see claims and examples * | 1-15 | |

-/--

The present search report has been drawn up for all claims

| Place of search | Date of completion of the search | Examiner |
|---|---|---|
| Munich | 11 March 2024 | Merckling-Ruiz, V |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

| Application Number |
| --- |
| EP 23 38 2662 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| Y | WO 2020/106591 A1 (EDWARDS STEVEN J [US]) 28 May 2020 (2020-05-28) * see claims and page 6 * ----- | 1-15 | |
| Y | WO 2018/165576 A1 (BIOHM HEALTH LLC [US]) 13 September 2018 (2018-09-13) * the whole document * ----- | 1-15 | |

**TECHNICAL FIELDS SEARCHED (IPC)**

The present search report has been drawn up for all claims

| Place of search | Date of completion of the search | Examiner |
| --- | --- | --- |
| Munich | 11 March 2024 | Merckling-Ruiz, V |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

           .......................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 23 38 2662

This annex lists the patent family members relating to the patent documents cited in the above-mentioned European search report.
The members are as contained in the European Patent Office EDP file on
The European Patent Office is in no way liable for these particulars which are merely given for the purpose of information.

11-03-2024

| Patent document cited in search report | | Publication date | Patent family member(s) | | Publication date |
|---|---|---|---|---|---|
| ES 2783885 | A1 | 18-09-2020 | ES | 2783723 A1 | 17-09-2020 |
| | | | ES | 2783885 A1 | 18-09-2020 |
| US 2022273735 | A1 | 01-09-2022 | EP | 4299067 A1 | 03-01-2024 |
| | | | TW | 202233217 A | 01-09-2022 |
| | | | US | 2022273735 A1 | 01-09-2022 |
| | | | WO | 2022180587 A1 | 01-09-2022 |
| WO 2019125204 | A1 | 27-06-2019 | RU | 2017145068 A | 21-06-2019 |
| | | | WO | 2019125204 A1 | 27-06-2019 |
| WO 2020106591 | A1 | 28-05-2020 | CA | 3120517 A1 | 28-05-2020 |
| | | | US | 2020155447 A1 | 21-05-2020 |
| | | | US | 2021299037 A1 | 30-09-2021 |
| | | | WO | 2020106591 A1 | 28-05-2020 |
| WO 2018165576 | A1 | 13-09-2018 | AU | 2018230495 A1 | 03-10-2019 |
| | | | BR | 112019018738 A2 | 07-04-2020 |
| | | | CA | 3055915 A1 | 13-09-2018 |
| | | | EP | 3592370 A1 | 15-01-2020 |
| | | | JP | 7189899 B2 | 14-12-2022 |
| | | | JP | 2020510084 A | 02-04-2020 |
| | | | JP | 2023014337 A | 26-01-2023 |
| | | | US | 2019381119 A1 | 19-12-2019 |
| | | | US | 2022296662 A1 | 22-09-2022 |
| | | | WO | 2018165576 A1 | 13-09-2018 |

EPO FORM P0459

For more details about this annex : see Official Journal of the European Patent Office, No. 12/82

**EP 4 483 889 A1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 3405563 A

- WO 2019025599 A1

**Non-patent literature cited in the description**

- **DEO PN** ; **DESHMUKH R**. Oral Microbiome: Unveiling the Fundamentals. *J Oral Maxillofac Pathol.*, 2019, vol. 23 (1), 122

- **OJEDA-GARCÉS** ; **JUAN CARLOS** ; **OVIEDO-GARCIA, ELIANA** ; **SALAS, LUIS ANDRÉS**. Streptococcus mutans and dental caries. *CES Dentistry*, 2013, vol. 26 (1), 44-56